# EUROPEAN PATENT APPLICATION

(11) **EP 4 817 428 A1**
(43) Date of publication of application: **07.10.2026**
(21) Application number: 24895193.1
(22) Date of filing: 29.11.2024
(51) Int. Cl.: A23L 27/00, A23C 9/156, A23L 2/38, A23L 11/65, A23L 27/20, A23L 27/50, C12G 3/04, C12G 3/08, C12G 3/022, C12N 1/14

(54) **SWEETNESS ENHANCER CONTAINING ERGOTHIONEINE AS ACTIVE INGREDIENT, AND FOOD OR BEVERAGE PRODUCT CONTAINING SAME**

(30) Priority: 29.11.2023 JP 2023201277; 29.11.2023 JP 2023201281
(71) Applicant: Kikkoman Corporation, Noda-shi, Chiba 278-8601 (JP)
(72) Inventor: ICHIKAWA, Keiichi, Noda-shi, Chiba 278-8601 (JP); TAJIMA, Ryoko, Noda-shi, Chiba 278-8601 (JP)
(74) Representative: TBK
(86) International application number: PCT/JP2024/042320
(87) International publication number: WO 2025/116010

(57) **Abstract**

Provided is a sweetness enhancer capable of enhancing sweetness of a food or beverage product without increasing a content of sugar-based sweet components such as sucrose, glucose, and fructose, or non-sugar sweet components. In addition, provided is a food or beverage product, such as food, beverage, seasoning, and alcoholic beverage, containing the sweetness enhancer and having enhanced sweetness due to a sweetness-enhancing effect thereof.

The sweetness enhancer for a food or beverage product containing a sweet component contains ergothioneine as an active ingredient, and a food or beverage product having enhanced sweetness is obtained by incorporating ergothioneine as a sweetness enhancer.

## Description

### TECHNICAL FIELD

The present invention relates to a sweetness enhancer for a food or beverage product, and a food or beverage product such as food, beverage, and seasoning in which sweetness is enhanced, containing the sweetness enhancer.

### BACKGROUND ART

Sweetness is a taste that is particularly widely preferred among five basic tastes (bitterness, saltiness, sourness, sweetness, and umami), but because the sweetness is not perceived as strongly as bitterness or saltiness, excessive intake of high-calorie sugars such as sucrose (sugar) and glucose (grape sugar), which are abundantly contained in food or beverage products, has become a global health problem. Against such a background and in view of recent health consciousness, although there is a strong demand for measures to avoid excessive intake of sugars, there arises a problem in that merely reducing sugar or the like in a food or beverage product affects the taste of the food or beverage product and reduces palatability.

Although low-calorie or non-calorie sweeteners have been widely used as sugar substitute sweeteners, food or beverage products using these high-intensity sweeteners often have inferior taste quality as compared with food or beverage products using sugar or the like. Because unpleasant bitterness or harshness is perceived, and the sweetness lasting long results in poor cleanness of taste. Accordingly, research and development have been conducted on sweetness-enhancing substances that enhance the sweetness of sugar or sugar substitute sweetener, and make it possible to maintain the sweetness and inherent palatability of a food or beverage product even when the amount of sugar substitute sweetener used is made as small as possible or the amount of sugar is reduced.

As such a sweetness-enhancing substance, Patent Literature 1 discloses methyl anthranilate, and it has been reported that, when the methyl anthranilate is added to a sucrose-containing food or beverage product having a sucrose concentration of 2% by mass, the methyl anthranilate exhibits a sweetness-enhancing effect corresponding to an increase of 0.4% to 0.6% by mass in the sucrose concentration of the food or beverage product. In addition, Patent Literature 2 discloses a specific quinoline-based compound, Patent Literature 3 discloses 2-(3-benzyloxypropyl)pyridine, and Patent Literature 4 discloses 1-(2-hydroxyphenyl)-3-(pyridin-4-yl)propan-1-one; however, all of these sweetness-enhancing substances are chemically synthesized substances, and their safety as foods has not been confirmed.

In addition, alcoholic beverages obtained by saccharification and/or brewing using koji, such as sake, shochu, mirin, and amazake, are produced from grains, grain koji, and water as raw materials, and thus contain only sweet components produced by decomposition and saccharification of the starch of the grains. Accordingly, various measures have been taken to increase the sugar content of alcoholic beverages and improve the sweetness thereof. For example, there have been reported a production method including a step of roasting only the raw grain material for kake used in alcoholic beverages with hot air at 200 to 400°C for several seconds to 5 minutes (Patent Literature 5), and a method in which, in order to efficiently increase the sugar content of amazake, a first step of mixing water with steamed rice and then adding rice koji to cause saccharification and fermentation and a second step of further adding rice koji to the saccharified and fermented steamed rice to cause saccharification and fermentation are repeated two or more times (Patent Literature 6). However, there arises a problem in that increasing the sugar content also increases a sticky mouthfeel.

### Prior Art Documents

### [Patent Literatures]

Patent Literature 1: Japanese Patent No. 6931289
Patent Literature 2: PCT Japanese Translation Patent Publication No. 2013-525278
Patent Literature 3: Japanese Patent Application Laid-Open No. 2016-202017
Patent Literature 4: PCT Japanese Translation Patent Publication No. 2011-512790
Patent Literature 5: Japanese Examined Patent Application, Second Publication No. H5-28591
Patent Literature 6: Japanese Patent Application Laid-Open No. 2011-55749
Patent Literature 7: Japanese Patent No. 6263672
Patent Literature 8: PCT International Publication No. WO2019/240243
Patent Literature 9: PCT International Publication No. WO2023/234307

### [Non-Patent Literatures]

Non-Patent Literature 1: BIOSCIENCE BIOTECHNOLOGY AND BIOCHEMISTRY (2019) Vol. 83, No. 1, p. 181-184
Non-Patent Literature 2: Journal of Bioscience and Bioengineering (2017) Vol. 124, No. 2, p. 178-183

### SUMMARY

### Technical Problem

An object of the present invention is to provide a sweetness enhancer capable of enhancing sweetness of a food or beverage product without increasing a content of sugar-based sweet components or non-sugar sweet components, which are sweetness components. Another object of the present invention is to provide a food or beverage product, such as a food, beverage, and seasoning, containing the sweetness enhancer and having enhanced sweetness due to a sweetness-enhancing effect thereof, and also to provide an alcoholic beverage having enhanced sweetness, the alcoholic beverage being obtained by saccharification and/or brewing of raw grains using koji.

### Solution to Problem

As a result of intensive studies on substances capable of enhancing sweetness of high-calorie carbohydrates such as sugar, the present inventors have found that, when ergothioneine, which can be produced only by certain microorganisms such as fungi including mushrooms and koji molds, actinomycetes, and cyanobacteria, is contained in a trace amount in a food or beverage product containing sugar-based sweet components such as sucrose, glucose, and fructose, the sweetness of the food or beverage product can be enhanced, and that the sweetness of alcoholic beverages obtained by saccharification and/or brewing of raw grains using koji can also be enhanced. The present inventors have further found that the ergothioneine can also enhance sweetness of non-sugar sweet components, thereby completing the present invention.

The present invention relates to a sweetness enhancer according to (1) to (3) below, a food or beverage product according to (4) to (9), (11), and (13) below, an amazake according to (10) below, or a mirin according to (12) below.
(1) A sweetness enhancer containing ergothioneine as an active ingredient.
(2) A sweetness enhancer for a food or beverage product containing a sugar-based sweet component, containing ergothioneine as an active ingredient.
(3) The sweetness enhancer according to (2), in which the sugar-based sweet component is one or more selected from the group consisting of sucrose (sugar), glucose (dextrose), fructose, fructose-glucose syrup, oligosaccharides, starch syrup, trehalose, maltose, and sugar alcohols such as sorbitol, mannitol, and xylitol.
(4) A food or beverage product having enhanced sweetness and containing a sugar-based sweet component, containing ergothioneine as a sweetness enhancer in an amount of 0.0001% by weight or more relative to a content of sucrose, 0.0001% by weight or more relative to a content of glucose, or 0.0003% by weight or more relative to a content of fructose.
(5) The food or beverage product according to (4), in which the food or beverage product is a food, a beverage, or a seasoning.
(6) The food or beverage product according to (4), in which the food or beverage product is not a soy sauce for retort food, a retort food containing soy sauce, a beverage having enhanced sourness, or a food or beverage product containing an ergothioneine-producing fungal fermentation biomass colored with a pigment or a supernatant thereof.
(7) The food or beverage product according to any one of (4) to (6), in which the food or beverage product is liquid sugar, prepared soy milk, milk, or an alcoholic beverage.
(8) The food or beverage product according to (7), in which the alcoholic beverage is sake, shochu, mirin, or amazake obtained by saccharification and/or brewing of raw grains using koji.
(9) The food or beverage product according to any one of (4) to (8), in which the food or beverage product is produced using a high ergothioneine-producing koji mold strain.
(10) An amazake containing 30 ppm or more of ergothioneine.
(11) The food or beverage product according to (4), in which the food or beverage product is not amazake.
(12) A mirin containing 2.5 ppm or more of ergothioneine.
(13) The food or beverage product according to (4), in which the food or beverage product is not mirin.

In addition, the present invention relates to a sweetness enhancer according to (14) and (15) below, a food or beverage product according to (16) and (17) below, or a method for enhancing sweetness according to (18) below.
(14) A sweetness enhancer for a food or beverage product containing a non-sugar sweet component, containing ergothioneine as an active ingredient.
(15) The sweetness enhancer according to (14), in which the non-sugar sweet component is one or more selected from the group consisting of high-intensity sweeteners including aspartame, acesulfame potassium, sucralose, stevia, luo han guo, licorice, and sweet amino acids.
(16) A food or beverage product having enhanced sweetness and containing a non-sugar sweet component, containing ergothioneine as a sweetness enhancer in an amount of 0.0005% by weight or more relative to a content of a high-intensity sweetener, or 0.0004% by weight or more relative to a content of a sweet amino acid.
(17) The food or beverage product according to (16), in which the food or beverage product is not a soy sauce for retort food, a retort food containing soy sauce, a beverage having enhanced sourness, or a food or beverage product containing an ergothioneine-producing fungal fermentation biomass colored with a pigment or a supernatant thereof.
(18) A method for enhancing sweetness of a food or beverage product, including incorporating ergothioneine into the food or beverage product.

In addition, the present invention relates to use of ergothioneine according to (19) to (23) below.
(19) Use of ergothioneine for enhancing sweetness of a food or beverage product.
(20) Use of ergothioneine for enhancing sweetness of a food or beverage product containing a sugar-based sweet component.
(21) The use of ergothioneine for enhancing sweetness according to (20), in which the sugar-based sweet component is one or more selected from the group consisting of sucrose (sugar), glucose (dextrose), fructose, fructose-glucose syrup, oligosaccharides, starch syrup, trehalose, maltose, and sugar alcohols such as sorbitol, mannitol, and xylitol.
(22) Use of ergothioneine for enhancing sweetness of a food or beverage product containing a non-sugar sweet component.
(23) The use of ergothioneine for enhancing sweetness according to (22), in which the non-sugar sweet component is one or more selected from the group consisting of high-intensity sweeteners including aspartame, acesulfame potassium, sucralose, stevia, luo han guo, licorice, and sweet amino acids.

### Advantageous Effects of Invention

According to the sweetness enhancer of the present invention, sweetness can be brought out while reducing the content of the sweet component in the food or beverage product by adding a very small amount thereof, so that the sweetness can be naturally enhanced without impairing the flavor. In addition, by eating or drinking a food or beverage product such as a seasoning, food, and beverage to which the sweetness enhancer is added, the intake of saccharides can be reduced, and excessive intake of sugars can be prevented.

In addition, since the inherent sweetness of the alcoholic beverage can be brought out without the need to increase the sugar content of the alcoholic beverage, the sweetness can be naturally enhanced without impairing the flavor. Furthermore, when the alcoholic beverage is mirin, addition of ergothioneine enhances not only sweetness but also umami. In addition, in the case of amazake produced using a high ergothioneine-producing koji mold strain, not only is the sweetness enhanced, but also the koji odor (3-octanol) is reduced to improve the aroma, and the mouthfeel becomes smoother and the amazake becomes easier to drink because fewer particles have large particle diameters.

### DESCRIPTION OF EMBODIMENTS

The present invention relates to a sweetness enhancer for a food or beverage product containing a sugar-based sweet component and/or a non-sugar sweet component, the sweetness enhancer containing ergothioneine as an active ingredient, and to a food or beverage product having enhanced sweetness, containing ergothioneine which is a sweetness enhancer.

In the present invention, "%" means "% by weight" even when the unit is not explicitly specified, and 1 ppm is 0.0001% by weight.

Ergothioneine (hereinafter also referred to as "ERG") is discovered as a sulfur-containing amino acid isolated from the rye ergot fungus (Claviceps purpurea), and has been confirmed to be present also in vivo in plants and animals. However, plants and animals cannot synthesize the ergothioneine, and ergothioneine in vivo is considered to be derived from ergothioneine synthesized by microorganisms such as basidiomycetes. Ergothioneine is known to be contained in some mushrooms of basidiomycetes, for example, edible mushrooms such as oyster mushrooms, shiitake mushrooms, maitake mushrooms, and king oyster mushrooms, and is particularly abundant in golden oyster mushrooms (Tamogitake). It is also known that koji molds produce the ERG.

Regarding the ERG, a biosynthesis pathway from histidine in microorganisms is known, and a sulfur atom is supplied from cysteine. The ERG has high antioxidant properties, and has been reported to have an elastase inhibitory effect and a tyrosinase inhibitory effect, attracting particular attention in the cosmetic and food fields such as skin whitening and wrinkle prevention. In addition, it has been found that the ERG is involved in the biological antioxidant defense system, and its application in the medical field is also being attempted. The ERG has high thermal stability and pH stability, and retains its antioxidant activity even at high temperatures. In addition to being incorporated into foods for expected physiological effects, the ERG is also expected to be used as an antioxidant in foods.

As methods for producing the ERG, extraction from basidiomycetes such as golden oyster mushrooms, chemical synthesis, and fermentation using microorganisms are carried out. The extraction from basidiomycetes such as golden oyster mushrooms takes time to obtain raw materials and is not suitable for mass production. Chemical synthesis is suitable for mass production, but requires the use of expensive synthesis reagents. Therefore, research has been conducted on fermentation-based production methods, such as fermentation using C1 compound-assimilating bacteria or yeast, fermentation using microorganisms engineered to overexpress ERG biosynthetic genes (Patent Literature 7), and solid culture using koji molds into which ERG biosynthetic genes have been introduced so as to overexpress ERG (Non-Patent Literature 1).

For the sweetness enhancer according to the embodiment of the present invention, a commercially available ERG product by chemical synthesis may be used, or an extract from mushrooms containing ERG, a koji mold producing ERG and a culture thereof, or sake lees produced using a koji mold, or a purified product thereof may be used. In addition, for a food or beverage product using koji mold as a raw material, the sweetness can be enhanced by increasing the amount of ERG in the food or beverage product by using a koji mold that highly produces ERG, without adding ERG.

As the mushrooms used for the extraction of ERG, mushrooms belonging to the genus Pleurotus of the family Pleurotaceae, such as golden oyster mushroom (Pleurotus cornucopiae var. citrinopileatus), oyster mushroom (Pleurotus ostreatus), shiitake mushroom (Lentinula edodes), and maitake mushroom (Grifola frondosa), can be preferably mentioned because of their high ERG content, and one or a combination of two or more of these can be used.

As the koji mold that produces ERG, any fungus belonging to the genus Aspergillus can be used. Examples thereof include Aspergillus oryzae, Aspergillus sojae, Aspergillus niger, Aspergillus luchuensis, and Aspergillus tamarii. As the strain of the koji mold, for example, a strain available from a depository institution such as Aspergillus oryzae RIB326 strain, a strain included in a commercially available seed koji, or a strain isolated and obtained from a food or beverage production environment such as a sake or soy sauce brewery can be used.

In addition, as the koji mold that produces ERG, in addition to the above-described wild strains, a mutant strain highly producing ERG can be isolated and used by a general mutagenesis method. Examples of the mutagenesis method include treatment with ultraviolet (UV) or X-ray irradiation that physically damages DNA to introduce mutations, and treatment with alkylating agents such as N-methyl-N'-nitro-N-nitrosoguanidine (NTG) and ethyl methanesulfonate (EMS) that chemically damage DNA to introduce mutations.

Furthermore, a high ERG-producing koji mold strain into which a gene related to ERG biosynthesis is introduced by genetic recombination can be used. Examples thereof include, but are not limited to, a koji mold mutant strain into which the egtA gene has been introduced (see

Patent Literature 8 and Non-Patent Literature 1), and a mutant strain of the genus Aspergillus in which ERG expression has been increased by introducing the AO090005000664 gene specific to the genus Aspergillus (amino acid sequence encoded thereby: SEQ ID NO: 1), or an ortholog gene thereof, into which a specific genetic mutation has been introduced, such as a G428S mutation, a C459F mutation, and a deletion of 1 to 112 amino acids in the region from W404 to Q515 (see Patent Literature 9).

As used herein, the term "high ERG-producing koji mold strain" refers to a strain in which the produced ERG is 5 to 10 times or more, preferably 20 times or more, in a liquid culture at 20 to 30°C for 3 to 14 days (the liquid medium is a 5% Puffmin SM (manufactured by Kikkoman Corporation) medium, with no pH adjustment) as compared to a non-mutated or non-transformed strain. Examples thereof include an Aspergillus sojae strain in which the above-described egtA gene is forcibly expressed, or an Aspergillus oryzae mutant strain containing an amino acid mutation or amino acid deletion in the region of W404 to Q515 of the above-described mutant AO090005000664 gene and an ortholog gene thereof.

When the food or beverage product is a product using a koji mold fermented product as a raw material, for example, soy sauce, sake, sweet sake (amazake), mirin, miso, or the like, the ERG content in the food or beverage product can be increased by producing koji using a high ERG-producing koji mold strain, even without adding ERG during production. In particular, when a high ERG-producing koji mold strain is used for producing a fermented food, a fermented beverage, or a fermented seasoning, effects other than sweetness enhancement are exhibited as compared to the case where ERG is added.

Alcoholic beverages using a koji mold fermented product as a raw material include sake, shochu, mirin, amazake, an alcohol-containing sweet seasoning, a fermented seasoning, a mirin-like seasoning, and the like. In addition, the amazake according to the embodiment of the present invention also includes rice koji amazake containing no alcohol.

Although, in addition to conventional raw material processing including polishing, washing, soaking, and steaming of brown rice, the use of kakemai subjected to a roasting treatment as a raw material is permitted, the raw materials for sake are limited to rice, rice koji, and water.

The production of sake consists of raw material processing including a koji-making step, mashing, fermentation, pressing, and refining, and the production of mirin consists of raw material processing, mashing, saccharification, aging, pressing, and refining. In addition, the production of amazake consists of raw material processing, mashing, and saccharification.

The amazake is a traditional Japanese sweet beverage made from rice, and known examples thereof include rice koji amazake obtained by saccharifying and decomposing rice with rice koji, amazake obtained by dissolving sake lees and adjusting the taste with sugar, and mixtures thereof. The rice koji amazake is usually produced by adding rice koji to steamed rice and keeping the mixture warm at a temperature of approximately 50°C, thereby subjecting the starch contained in the polished rice to saccharification fermentation in which glucose is produced by decomposition with saccharifying enzymes, and contains no alcohol. The amazake using sake lees as a raw material contains less than 1% of alcohol. The amazake according to the embodiment of the present invention also includes amazake containing no alcohol. It has been reported (Non-Patent Literature 2) that the ergothioneine content of commercially available amazake is less than 10 ppm even in products having high ergothioneine contents.

In addition, the mirin is a type of seasoning widely used in cooking, and is produced by mixing rice materials such as steamed glutinous rice with rice koji and alcohol such as shochu, saccharifying the rice materials through the action of koji mold, causing components in the rice materials to be decomposed and dissolved out, and subjecting the moromi, after further aging, to treatments such as pressing, clarification, filtration, and pasteurization.

The sugar-based sweet component in the present invention includes sucrose (sugar), glucose (dextrose), fructose, fructose-glucose syrup, oligosaccharides, starch syrup, trehalose, maltose, sugar alcohols such as sorbitol, mannitol, and xylitol, maple syrup, honey, and the like.

In addition, the non-sugar sweet component in the present invention includes high-intensity sweeteners such as acesulfame potassium, aspartame, sucralose, stevia, luo han guo, and licorice, and sweet amino acids such as glycine (Gly), alanine (Ala), threonine (Thr), serine (Ser), lysine (Lys), and proline (Pro).

The food or beverage product according to the embodiment of the present invention contains one or more sugar-based sweet components and/or one or more non-sugar sweet components.

The enhancement of sweetness means that the sweetness provided by these components is perceived more strongly as compared to the case without the sweetness enhancer according to the embodiment of the present invention. Specifically, it means that, by adding the sweetness enhancer, equivalent sweetness can be perceived even when sugar or the like is reduced by, for example, 1% to 50%. It also means that, by adding the sweetness enhancer, sweetness equivalent to that in the case of containing, for example, 1.1 to 2 times the amount of sugar or the like can be perceived. Alternatively, when sugar or the like is contained in a small amount but cannot be perceived, it means that sweetness can be perceived for the first time by adding the sweetness enhancer.

The amount of ERG having a sweetness-enhancing effect relative to the sucrose content of the food or beverage product is 0.01% by weight or more of ERG for a sucrose solution of 0.5% or more and less than 1%, 0.0025% by weight or more for a sucrose solution of 1% or more and less than 10%, and 0.0001% by weight or more for a sucrose solution of 10% or more. Since almost no sweetness is perceived in a food or beverage product having a sucrose content of less than 1%, the sweetness-enhancing effect on the food or beverage product can generally be exerted by incorporating ERG in an amount of 0.0001% by weight or more, or 0.0025% by weight or more, relative to the sucrose content.

As described above, the sweetness enhancer according to the embodiment of the present invention exerts a sweetness-enhancing effect on a food or beverage product by incorporating ERG in an amount of 0.0001% by weight or more, preferably 0.0025% by weight or more, relative to the sucrose content in the food or beverage product.

In addition, for a food or beverage product containing glucose or fructose, the sweetness-enhancing effect on the food or beverage product is exerted by incorporating ERG in an amount of 0.0001% by weight or more, preferably 0.0005% by weight or more, relative to the glucose content, or by incorporating ERG in an amount of 0.0003% by weight or more relative to the fructose content. When the food or beverage product contains any one or more of sucrose, glucose, or fructose, the ERG may be incorporated in an amount of 0.0001 to 0.0003% by weight or more relative to any of the contents.

The sweetness enhancer according to the embodiment of the present invention exerts a sweetness-enhancing effect on a food or beverage product by incorporating ERG in an amount of 0.0005% by weight or more relative to the aspartame content in the food or beverage product.

In addition, for a food or beverage product containing stevia, the sweetness-enhancing effect on the food or beverage product is exerted by incorporating ERG in an amount of 0.05% by weight or more relative to the stevia content, or by incorporating ERG in an amount of 0.0004% by weight or more relative to the sweet amino acid content. When the food or beverage product contains a non-sugar sweet component, the ERG may be incorporated in an amount of 0.0004 to 0.0005% by weight or more relative to the non-sugar sweet component other than stevia.

In addition, since the sweetness-enhancing effect does not increase even when a large amount of the sweetness enhancer according to the embodiment of the present invention is incorporated, considering economic efficiency, the ERG can be incorporated into the food or beverage product in an amount of 0.15% by weight or less, for example, 0.12% or 0.09% by weight or less, or 0.06% or 0.03% by weight or less. Relative to the content of the sweet component in the food or beverage product, the ERG can be incorporated in an amount of 15% by weight or less, for example, 12% or 9% by weight or less, or 6% or 3% by weight or less.

The sweetness of the alcoholic beverage obtained by saccharification and/or brewing of raw grains using koji in the present invention is enhanced generally by containing ERG in an amount of 10 ppm or more, for example, 10 ppm or more, 15 ppm or more, 30 ppm or more, or 35 ppm or more. Among these, it is preferable that the amazake contains 30 ppm of ERG. In addition, since mirin is sweeter than other alcoholic beverages, the effect is exhibited when ERG is 2.5 ppm or more.

Furthermore, since the sweetness-enhancing effect does not necessarily increase even when a large amount of ERG is incorporated, considering economic efficiency, the ERG can be incorporated into the alcoholic beverage in an amount of 0.15% by weight or less, for example, 0.12% or 0.09% by weight or less, or 0.06% or 0.03% by weight or less.

The sweetness enhancer according to the embodiment of the present invention exerts a sweetness-enhancing effect on a food or beverage product at contents of all combinations of these lower limit values and upper limit values for various sweet components in the food or beverage product.

The concentration of ERG is measured by LCMS under the following conditions.

### (HPLC conditions)

HPLC analysis is performed under the following conditions.
Apparatus: HPLC apparatus; HPLC: Nexera series set (manufactured by Shimadzu Corporation)
Column: COSMOSIL 2.5HILIC column 3.0 x 150 2.5 µm 3.0 mm I.D. x 15.0 cm (manufactured by Nacalai Tesque, Inc.)
Flow rate: 0.5 mL/min
Temperature: 40°C
Mobile phase: A) 0.1% (v/v) aqueous formic acid solution, B) 0.1 (v/v) formic acid in acetonitrile
Isocratic: 0 to 10 minutes (B: 80%)
Injection volume: 5 µL

### (Mass spectrometry)

Apparatus: LCMS-2020 (manufactured by Shimadzu Corporation)
(Mass spectrometry (LC-MS) analysis conditions)
Ionization conditions: ESI+
MS conditions: SIM ERG; m/z: 230.1 ([M+H]⁺)
Retention time: around 5 minutes

The food or beverage product containing a sugar-based sweet component and/or a non-sugar sweet component according to the embodiment of the present invention is food, beverage, or seasoning.

The food can be used for any food and is not limited. Examples thereof include frozen desserts such as ice cream and sherbet; desserts such as jelly, pudding, and yokan; dairy products or dairy substitutes such as yogurt, cheese, and whipped cream; spreads such as nut butter; baked confectionery products such as cookies, biscuits, and cakes; confectioneries such as chocolate, chewing gum, and manju; breads such as sweet buns and loaf bread; jams; ramune; tablets; tablet confectioneries; mycoprotein; pet food; and medical foods.

The beverage is also not limited, and examples thereof include soft drinks, milk beverages, soups for drinking including canned soups, soy milk beverages such as prepared soy milk, sports drinks, fruit juice beverages, alcoholic beverages such as sake, shochu, and cocktails, amazake which is a saccharified rice product, black tea, coffee, green tea, cocoa, and nutritional drinks classified as quasi-drugs. The food or beverage is not limited to liquid forms, and also includes gel-like and semi-solid forms such as jelly beverages.

The seasoning is also not limited, and examples thereof include soy sauce, mirin, miso, cooking sake, sauces, tsuyu, broths such as bonito stock, sushi vinegar, tare, ketchup, liquid sugar, and syrup.

### Examples

Hereinafter, the present invention will be specifically described in detail with reference to Examples, but the present invention is not limited thereto.

In Examples, when simply indicated as "%", it means "% by weight". In addition, samples at room temperature were used for sensory evaluation.

### [Test 1: Sweetness-enhancing effect of sucrose solution by ERG]

Sucrose was added to pure water to prepare sucrose samples having six concentrations of 0%, 1%, 1.5%, 2%, 2.5%, and 3%; and as a seventh sample, a sample in which purified ergothioneine (purity: 99.5% or more, Tetrahedron) was added to 2% sucrose to a final concentration of 60 ppm was prepared.

The sweetness was evaluated by a sensory evaluation by an expert panel consisting of five trained sweetness-sensitive subjects. The panel of five experts was asked to select a sample having an equivalent sweetness from among the 1st to 6th sucrose samples (the concentration was not disclosed to the panel) regarding the sweetness of the 7th 2% sucrose sample containing 60 ppm ERG. After tasting each sample and before tasting the next sample, the mouth was completely rinsed with water at room temperature.

As a result, all the five panelists evaluated the 7th 2% sucrose sample containing ERG to have sweetness equivalent to that of the 2.5% sucrose solution. By the addition of 60 ppm of ERG, the sweetness of the 2% sucrose solution became equivalent to that of the 2.5% sucrose solution, and the sweetness-enhancing effect of ERG was confirmed.

### [Test 2: Confirmation of sweetness of ERG itself]

In the same manner as in Test 1, sucrose samples having six concentrations of 0%, 1%, 1.5%, 2%, 2.5%, and 3%, and as a seventh sample, a sample in which ergothioneine was added to water to a final concentration of 60 ppm were prepared. A drinking test by the same expert panel of five as in Test 1 was conducted, and after tasting each sample and before tasting the next sample, the mouth was completely rinsed with water at room temperature, and sweetness was sensorially evaluated. The panel of five experts was asked to select a sample having an equivalent sweetness from among the 1st to 6th sucrose samples (the concentration was not disclosed to the panel) regarding the sweetness of the 7th sample.

As a result, all the five panelists evaluated the 7th sample (ERG added to water to a final concentration of 60 ppm) to have sweetness equivalent to that of the 0% sucrose solution, and it was confirmed that ERG itself has no sweetness.

### [Test 3: ERG addition amount for enhancing sweetness of sucrose]

### (1) Case of 1% to 50% sucrose samples

Samples were prepared by adding ergothioneine to 1% to 50% sucrose solutions to final concentrations of 0 ppm, 0.05 ppm, 0.1 ppm, 0.25 ppm, and 0.5 ppm (ERG0 to 0.5 in Table 1 below; hereinafter, "ERGX" means a sample to which X ppm of ERG is added).

**[Table 1]**

| | ERG0 | ERG0.05 | ERG0.1 | ERG0.25 | ERG0.5 |
|---|---|---|---|---|---|
| Sucrose | 1% to 50% | | | | |
| ERG | 0 ppm | 0.05 ppm | 0.1 ppm | 0.25 ppm | 0.5 ppm |

A drinking test by an expert panel of four, different from those in Tests 1 and 2, was conducted on 1% to 50% sucrose samples to which ERG was added at each concentration (the concentration was not disclosed to the panel), using a 1% to 50% sucrose sample to which no ergothioneine was added as a control, and sweetness was sensorially evaluated. After tasting each sample and before tasting the next sample, the mouth was completely rinsed with water at room temperature. Pairs combining the control 1% to 50% sucrose sample and the 1% to 50% sucrose sample containing ergothioneine at each concentration were presented to the expert panel, and the panel evaluated which sample of the presented pair was perceived to be sweeter by a paired comparison test.

The evaluation results are shown in Table 2.

### <Evaluation method of paired comparison test>

Evaluation results of sweetness of test product compared with control
∘: Sweetness was enhanced compared to the control.
◇: Sweetness was similar to that of the control.

The evaluation method of the paired comparison test and the indication of the evaluation results are the same in other tests.

**[Table 2]**

| Panelist A | | | | | |
|---|---|---|---|---|---|
| Concentration of sucrose | ERG0 | ERG0.05 | ERG0.1 | ERG0.25 | ERG0.5 |
| 0.5% | ◊ | ◊ | ◊ | ◊ | ○ |
| 1% | ◊ | ◊ | ◊ | ○ | ○ |
| 2% | ◊ | ◊ | ◊ | ○ | ○ |
| 5% | ◊ | ◊ | ◊ | ○ | ○ |
| 10% | ◊ | ◊ | ◊ | ○ | ○ |
| 15% | ◊ | ◊ | ○ | ○ | ○ |
| 20% | ◊ | ◊ | ○ | ○ | ○ |
| 30% | ◊ | ◊ | ○ | ○ | ○ |
| 40% | ◊ | ◊ | ○ | ○ | ○ |
| 50% | ◊ | ◊ | ○ | ○ | ○ |

| Panelist B | | | | | |
|---|---|---|---|---|---|
| Concentration of sucrose | ERG0 | ERG0.05 | ERG0.1 | ERG0.25 | ERG0.5 |
| 0.5% | ◊ | ◊ | ◊ | ○ | ○ |
| 1% | ◊ | ◊ | ◊ | ○ | ○ |
| 2% | ◊ | ◊ | ○ | ○ | ○ |
| 5% | ◊ | ○ | ○ | ○ | ○ |
| 10% | ◊ | ○ | ○ | ○ | ○ |
| 15% | ◊ | ○ | ○ | ○ | ○ |
| 20% | ◊ | ○ | ○ | ○ | ○ |
| 30% | ◊ | ○ | ○ | ○ | ○ |
| 40% | ◊ | ○ | ○ | ○ | ○ |
| 50% | ◊ | ○ | ○ | ○ | ○ |

| Panelist C | | | | | |
|---|---|---|---|---|---|
| Concentration of sucrose | ERG0 | ERG0.05 | ERG0.1 | ERG0.25 | ERG0.5 |
| 0.5% | ◊ | ◊ | ◊ | ○ | ○ |
| 1% | ◊ | ◊ | ◊ | ○ | ○ |
| 2% | ◊ | ◊ | ◊ | ○ | ○ |
| 5% | ◊ | ◊ | ○ | ○ | ○ |
| 10% | ◊ | ◊ | ○ | ○ | ○ |
| 15% | ◊ | ◊ | ○ | ○ | ○ |
| 20% | ◊ | ◊ | ○ | ○ | ○ |
| 30% | ◊ | ◊ | ○ | ○ | ○ |
| 40% | ◊ | ◊ | ○ | ○ | ○ |
| 50% | ◊ | ◊ | ○ | ○ | ○ |

| Panelist D | | | | | |
|---|---|---|---|---|---|
| Concentration of sucrose | ERG0 | ERG0.05 | ERG0.1 | ERG0.25 | ERG0.5 |
| 0.5% | ◊ | ◊ | ◊ | ○ | ○ |
| 1% | ◊ | ◊ | ◊ | ○ | ○ |
| 2% | ◊ | ◊ | ○ | ○ | ○ |
| 5% | ◊ | ◊ | ○ | ○ | ○ |
| 10% | ◊ | ◊ | ○ | ○ | ○ |
| 15% | ◊ | ◊ | ○ | ○ | ○ |
| 20% | ◊ | ◊ | ○ | ○ | ○ |
| 30% | ◊ | ◊ | ○ | ○ | ○ |
| 40% | ◊ | ◊ | ○ | ○ | ○ |
| 50% | ◊ | ◊ | ○ | ○ | ○ |

Table 2 shows the evaluation results of the paired comparison test by four expert panelists A to D, and indicates the lower limit of the ERG concentration for the sweetness-enhancing effect on the 1% to 50% sucrose solution.

For the sucrose solution of 0.5% or more and less than 1%, when 0.5 ppm or more of ERG was added, all evaluated that the sweetness was enhanced. For the sucrose solution of 1% or more and less than 10%, when 0.25 ppm or more of ERG was added, all evaluated that the sweetness was enhanced. For the sucrose solution of 10% or more, when 0.1 ppm or more of ERG was added, all evaluated that the sweetness was enhanced.

The amount of ERG having a sweetness-enhancing effect relative to the sucrose content of the food or beverage product corresponds to 0.01% by weight or more of ERG for a sucrose solution of 0.5% or more and less than 1%, 0.0025% by weight or more for a sucrose solution of 1% or more and less than 10%, and 0.0001% by weight or more for a sucrose solution of 10% or more.

### (2) Case of 4% sucrose sample

Samples (ERG5 to 900) were prepared by adding ergothioneine to a 4% sucrose solution to final concentrations of 5 ppm, 20 ppm, 60 ppm, 100 ppm, 400 ppm, and 900 ppm, and a drinking test by an expert panel of four, different from those in Tests 1 and 2, was conducted on the 4% sucrose samples to which ERG was added at each concentration (the concentration was not disclosed to the panel), and sweetness was sensorially evaluated.

Pairs combining the control 4% sucrose sample of Table 3 below and the 4% sucrose sample containing ergothioneine at each concentration were presented to the expert panel, and the panel evaluated which sample of the presented pair was perceived to be sweeter by a paired comparison test.

**[Table 3]**

| | | Panelist | | | |
|---|---|---|---|---|---|
| Control | Test product | A | B | C | D |
| ERG5 | ERG20 | ○ | ○ | ○ | ○ |
| ERG20 | ERG60 | ○ | ○ | ○ | ○ |
| ERG60 | ERG100 | ○ | ○ | ○ | ○ |
| ERG100 | ERG400 | ○ | ◊ | ◊ | ◊ |
| ERG400 | ERG900 | ◊ | ◊ | ◊ | ◊ |

Table 3 shows the ERG concentration at which the sweetness-enhancing effect on 4% sucrose becomes constant.

Since the 4% sucrose sample to which 400 ppm of ERG was added was evaluated to have the same degree of sweetness as that to which 100 ppm of ERG was added, it was evaluated that, even when ERG was added in an amount exceeding 100 ppm, the degree of sweetness enhancement did not change from that to which 100 ppm was added, and it was confirmed that the sweetness-enhancing effect became constant at 100 ppm of ERG.

From the above, for the 4% sucrose solution, it was evaluated that, when ERG was added at 5 ppm to 100 ppm, the sweetness was sequentially enhanced as the addition amount increased. When it exceeded 100 ppm, the sweetness-enhancing effect did not differ from that with the addition of 100 ppm.

### [Test 4: Upper limit of ERG addition amount]

ERG was dissolved in water at 40°C so that the ergothioneine concentration became 10% (100,000 ppm), and then left to stand at 4°C. When the solution was checked 1 day later, ERG was precipitated. From the above, the concentration at which ERG could be contained in water was less than 10%.

### [Test 5: Sweetness-enhancing effect of glucose solution by ERG]

Glucose was added to pure water to prepare glucose samples having six concentrations of 0%, 13%, 14%, 15%, 16%, and 17%; and as a seventh sample, a sample in which ergothioneine was added to 15% glucose to a final concentration of 60 ppm was prepared.

Sweetness was sensorially evaluated by the same expert panel of five trained sweetness-sensitive subjects as in Test 1. The panel of five experts was asked to select a sample having an equivalent sweetness from among the 1st to 6th glucose samples (the concentration was not disclosed to the panel) regarding the sweetness of the 7th 15% glucose sample containing 60 ppm ERG.

As a result, three of the five panelists evaluated the 7th 15% glucose sample containing ERG to have sweetness equivalent to that of the 16% glucose solution, and two evaluated it to have sweetness equivalent to that of the 17% glucose solution. By the addition of 60 ppm of ERG, the sweetness of the 15% glucose solution became equivalent to that of the 16% glucose solution, and the sweetness-enhancing effect of ERG on glucose was confirmed.

### [Test 6: ERG addition amount for enhancing sweetness of glucose]

### (1) Case of 50% glucose sample

Samples ERG0 to 900 were prepared by adding ergothioneine to 50% glucose samples to final concentrations of 0.5 ppm, 1 ppm, 3 ppm, 5 ppm, 10 ppm, 20 ppm, 60 ppm, 100 ppm, 400 ppm, and 900 ppm (Table 4).

**[Table 4]**

| | ERG0 | ERG0.5 | ERG1 | ERG3 | ERG5 | ERG10 | ERG20 | ERG60 | ERG100 | ERG400 | ERG900 |
|---|---|---|---|---|---|---|---|---|---|---|---|
| Glucose | 50% | 50% | 50% | 50% | 50% | 50% | 50% | 50% | 50% | 50% | 50% |
| ERG | 0 ppm | 0.5 ppm | 1 ppm | 3 ppm | 5 ppm | 10 ppm | 20 ppm | 60 ppm | 100 ppm | 400 ppm | 900 ppm |

Pairs combining a control 50% glucose sample to which ergothioneine was not added and a 50% glucose sample containing ergothioneine at each concentration were presented to the expert panel of four, and the panel evaluated which sample of the presented pair was perceived to be sweeter by a paired comparison test. The results are shown in Table 5.

**[Table 5]**

| | | Panelist | | | |
|---|---|---|---|---|---|
| Control | Test product | A | B | C | D |
| ERG0 | ERG0 | ◊ | ◊ | ◊ | ◊ |
| ERG0 | ERG0.5 | ○ | ○ | ○ | ○ |
| ERG0 | ERG1 | ○ | ○ | ○ | ○ |
| ERG0 | ERG3 | ○ | ○ | ○ | ○ |
| ERG0 | ERG5 | ○ | ○ | ○ | ○ |
| ERG0 | ERG10 | ○ | ○ | ○ | ○ |
| ERG0 | ERG20 | ○ | ○ | ○ | ○ |

Table 5 shows the lower limit of the ERG concentration for the sweetness-enhancing effect on the 50% glucose solution.

For the 50% glucose solution, when 0.5 ppm or more of ERG was added, all the panelists evaluated that the sweetness was enhanced. This corresponds to 0.0001% by weight or more of ERG having a sweetness-enhancing effect, based on the glucose content of the food or beverage product.

### (2) Case of 10% glucose solution

The same test as in the 50% glucose solution of (1) described above was conducted on a 10% glucose solution using the samples (ERG0 to 20) shown in Table 6. The results of sensory evaluation by the paired comparison test are shown in Tables 7 and 8.

**[Table 6]**

| | ERG0 | ERG0.5 | ERG1 | ERG3 | ERG5 | ERG10 | ERG20 |
|---|---|---|---|---|---|---|---|
| Glucose | 10% | 10% | 10% | 10% | 10% | 10% | 10% |
| ERG | 0 ppm | 0.5 ppm | 1 ppm | 3 ppm | 5 ppm | 10 ppm | 20 ppm |

**[Table 7]**

| | | Panelist | | | |
|---|---|---|---|---|---|
| Control | Test product | A | B | C | D |
| ERG0 | ERG0 | ◊ | ◊ | ◊ | ◊ |
| ERG0 | ERG0.25 | ◊ | ◊ | ◊ | ◊ |
| ERG0 | ERG0.5 | ○ | ○ | ○ | ○ |
| ERG0 | ERG1 | ○ | ○ | ○ | ○ |
| ERG0 | ERG3 | ○ | ○ | ○ | ○ |
| ERG0 | ERG5 | ○ | ○ | ○ | ○ |
| ERG0 | ERG10 | ○ | ○ | ○ | ○ |
| ERG0 | ERG20 | ○ | ○ | ○ | ○ |

Table 7 shows the lower limit of the ERG concentration for the sweetness-enhancing effect on the 10% glucose solution.

For the 10% glucose solution, when 0.5 ppm or more of ERG was added, all the panelists evaluated that the sweetness was enhanced. This corresponds to 0.0005% by weight or more of ERG having a sweetness-enhancing effect, based on the glucose content of the food or beverage product.

**[Table 8]**

| | | Panelist | | | |
|---|---|---|---|---|---|
| Control | Test product | A | B | C | D |
| ERG5 | ERG20 | ○ | ○ | ○ | ○ |
| ERG20 | ERG60 | ○ | ◊ | ○ | ○ |
| ERG60 | ERG100 | ◊ | ◊ | ○ | ◊ |
| ERG100 | ERG400 | ◊ | ◊ | ◊ | ◊ |
| ERG400 | ERG900 | ◊ | ◊ | ◊ | ◊ |

Table 8 shows the ERG concentration at which the sweetness-enhancing effect on the 10% glucose solution becomes constant.

Since the 10% glucose sample to which 100 ppm of ERG was added was evaluated to have the same degree of sweetness as that to which 60 ppm of ERG was added, it was evaluated that, even when ERG was added in an amount exceeding 60 ppm, the degree of sweetness enhancement did not change from that to which 60 ppm was added, and it was confirmed that the sweetness-enhancing effect became constant at 60 ppm of ERG.

From the above, for the 10% glucose solution, it was evaluated that, when ERG was added at 0.5 ppm to 60 ppm, the sweetness was sequentially enhanced as the addition amount increased. When it exceeded 60 ppm, the sweetness-enhancing effect did not differ from that with the addition of 60 ppm.

### [Test 7: Sweetness-enhancing effect of fructose solution by ERG]

Fructose was added to pure water to prepare fructose samples having six concentrations of 0%, 4.5%, 5%, 5.25%, 5.5%, and 6%; and as a seventh sample, a sample in which ergothioneine was added to 5% fructose to a final concentration of 60 ppm was prepared.

Sweetness was sensorially evaluated by the same expert panel of five trained sweetness-sensitive subjects as in Test 1. The panel of five experts was asked to select a sample having an equivalent sweetness from among the 1st to 6th fructose samples (the concentration was not disclosed to the panel) regarding the sweetness of the 7th 5% fructose sample containing 60 ppm ERG.

As a result, three of the five panelists evaluated the 7th sample to have sweetness equivalent to that of the 5.5% fructose solution, and two evaluated it to have sweetness equivalent to that of the 5.25% fructose solution. By the addition of 60 ppm of ERG, the sweetness of the 5% fructose solution became equivalent to that of the 5.25 to 5.5% fructose solution, and the sweetness-enhancing effect of ERG on fructose was confirmed.

### [Test 8: ERG addition amount for enhancing sweetness of fructose by ERG]

Samples (ERG0 to 10) were prepared by adding ergothioneine to 20% fructose samples to final concentrations of 0.5 ppm, 1 ppm, 3 ppm, 5 ppm, and 10 ppm.

A drinking test by a panel of four experts was conducted on 20% fructose samples to which ERG was added at each concentration (the concentration was not disclosed to the panel), using a 20% fructose sample to which no ergothioneine was added as a control, and sweetness was sensorially evaluated. The evaluation results are shown in Table 9.

In addition, samples (ERG0.5 to 900) were prepared by adding ergothioneine to 20% fructose samples to final concentrations of 0.5 ppm, 5 ppm, 10 ppm, 20 ppm, 40 ppm, 60 ppm, 80 ppm, 100 ppm, 200 ppm, 400 ppm, and 900 ppm.

Pairs combining 20% fructose samples containing different concentrations of ERG (for example, a pair of ERG 20 ppm and 40 ppm) were presented to the expert panel, and the panel evaluated which sample of the presented pair was perceived to be sweeter by a paired comparison test. The evaluation results are shown in Table 10.

**[Table 9]**

| | | Panelist | | | |
|---|---|---|---|---|---|
| Control | Test product | A | B | C | D |
| ERG0 | ERG0 | ◊ | ◊ | ◊ | ◊ |
| ERG0 | ERG0.5 | ○ | ○ | ○ | ○ |
| ERG0 | ERG1 | ○ | ○ | ○ | ○ |
| ERG0 | ERG3 | ○ | ○ | ○ | ○ |
| ERG0 | ERG5 | ○ | ○ | ○ | ○ |
| ERG0 | ERG10 | ○ | ○ | ○ | ○ |

**[Table 10]**

| | | Panelist | | | |
|---|---|---|---|---|---|
| Control | Test product | A | B | C | D |
| ERG0.5 | ERG5 | ○ | ○ | ○ | ○ |
| ERG10 | ERG20 | ○ | ○ | ○ | ○ |
| ERG20 | ERG40 | ○ | ○ | ○ | ○ |
| ERG40 | ERG60 | ◊ | ◊ | ○ | ◊ |
| ERG60 | ERG80 | ◊ | ◊ | ○ | ◊ |
| ERG80 | ERG100 | ◊ | ◊ | ◊ | ◊ |
| ERG100 | ERG200 | ◊ | ◊ | ◊ | ◊ |
| ERG200 | ERG400 | ◊ | ◊ | ◊ | ◊ |
| ERG400 | ERG900 | ◊ | ◊ | ◊ | ◊ |

For the 20% fructose samples to which 0.5 ppm or more of ERG was added, all the panelists evaluated that the sweetness was enhanced. This corresponds to 0.0003% by weight or more of ERG having a sweetness-enhancing effect, based on the fructose content of the food or beverage product.

In addition, since the 20% fructose sample to which 100 ppm of ERG was added was evaluated to have the same degree of sweetness as that to which 80 ppm of ERG was added, it was evaluated that, even when ERG was added in an amount exceeding 80 ppm, the degree of sweetness enhancement did not change from that to which 80 ppm was added, and it was confirmed that the sweetness-enhancing effect became constant at 80 ppm of ERG.

From the above, for the 20% fructose solution, it was evaluated that, when ERG was added at 0.5 ppm to 80 ppm, the sweetness was sequentially enhanced as the addition amount increased. When it exceeded 80 ppm, the sweetness-enhancing effect did not differ from that with the addition of 80 ppm.

### [Test 9: Addition amount of ERG for enhancing sweetness of various sweet components]

In the following Test 9, sensory evaluation was performed by a panel of three experts in the same paired comparison test as in Tests 3, 5, and 7.

### (1) Trehalose

Samples were prepared by adding ergothioneine to a 50% trehalose sample to final concentrations of 0.5 ppm, 1 ppm, 3 ppm, 5 ppm, 10 ppm, and 20 ppm (Table 11, ERG0 to 20).

**[Table 11]**

| | ERG0 | ERG0.5 | ERG1 | ERG3 | ERG5 | ERG10 | ERG20 |
|---|---|---|---|---|---|---|---|
| Trehalose | 50% | 50% | 50% | 50% | 50% | 50% | 50% |
| ERG | 0 ppm | 0.5 ppm | 1 ppm | 3 ppm | 5 ppm | 10 ppm | 20 ppm |

For the 50% trehalose samples to which ERG was added at each concentration (the concentration was not disclosed to the panel), pairs combining a control 50% trehalose sample and a 50% trehalose sample containing ergothioneine at each concentration were presented to the expert panel, and the panel evaluated which sample of the presented pair was perceived to be sweeter by a paired comparison test. The test results are shown in Table 12.

**[Table 12]**

| | | Panelist | | |
|---|---|---|---|---|
| Control | Test product | A | B | C |
| ERG0 | ERG0 | ◇ | ◇ | ◇ |
| ERG0 | ERG0.5 | ○ | ○ | ○ |
| ERG0 | ERG1 | ○ | ○ | ○ |
| ERG0 | ERG3 | ○ | ○ | ○ |
| ERG0 | ERG5 | ○ | ○ | ○ |
| ERG0 | ERG10 | ○ | ○ | ○ |
| ERG0 | ERG20 | ○ | ○ | ○ |

For the 50% trehalose samples to which 0.5 ppm or more of ERG was added, all the panelists evaluated that the sweetness was enhanced. This corresponds to 0.0001% by weight or more of ERG having a sweetness-enhancing effect, based on the trehalose content of the food or beverage product.

### (2) Fructose-glucose syrup

Samples were prepared by adding ERG to a 50% liquid sugar sample of an isomerized sugar containing 55% or more of fructose (New Fructo 55 (NF55), Showa Sangyo Co., Ltd.) to the ERG concentrations shown in Table 13 (ERG0 to 20). Pairs combining a control 50% liquid sugar sample and a 50% liquid sugar sample containing ERG at each concentration were presented, and the panel evaluated which sample of the presented pair was perceived to be sweeter by a paired comparison test. The test results are shown in Table 13.

**[Table 13]**

| | | Panelist | | | |
|---|---|---|---|---|---|
| Control | Test product | A | B | C | D |
| ERG0 | ERG0 | ◊ | ◊ | ◊ | ◊ |
| ERG0 | ERG0.5 | ○ | ○ | ○ | ○ |
| ERG0 | ERG1 | ○ | ○ | ○ | ○ |
| ERG0 | ERG3 | ○ | ○ | ○ | ○ |
| ERG0 | ERG5 | ○ | ○ | ○ | ○ |
| ERG0 | ERG10 | ○ | ○ | ○ | ○ |
| ERG0 | ERG20 | ○ | ○ | ○ | ○ |

For the 50% liquid sugar samples to which 0.5 ppm or more of ERG was added, all the panelists evaluated that the sweetness was enhanced.

### (3) Highly saccharified reduced starch syrup (sugar alcohol)

Samples were prepared by adding ERG to a 50% starch syrup sample of a highly saccharified reduced starch syrup rich in monosaccharide alcohols and disaccharide alcohols (SE 600, Bussan Food Science Co., Ltd.) to the ERG concentrations shown in Table 14 (ERG0 to 10). Pairs combining a control 50% starch syrup sample and a 50% starch syrup sample containing ERG at each concentration were presented, and the panel evaluated which sample of the presented pair was perceived to be sweeter by a paired comparison test. The test results are shown in Table 14.

**[Table 14]**

| | | Panelist | | |
|---|---|---|---|---|
| Control | Test product | A | B | C |
| ERG0 | ERG0 | ◇ | ◇ | ◇ |
| ERG0 | ERG0.5 | ○ | ○ | ○ |
| ERG0 | ERG1 | ○ | ○ | ○ |
| ERG0 | ERG3 | ○ | ○ | ○ |
| ERG0 | ERG5 | ○ | ○ | ○ |
| ERG0 | ERG10 | ○ | ○ | ○ |
| ERG0 | ERG20 | ○ | ○ | ○ |

For the 50% starch syrup samples to which 0.5 ppm or more of ERG was added, all the panelists evaluated that the sweetness was enhanced.

### (4) Oligosaccharide

Samples were prepared by adding ERG to a 50% oligosaccharide sample of an oligosaccharide (Fuji Oligo #450, Nihon Shokuhin Kako Co., Ltd.) to the ERG concentrations shown in Table 15 (ERG0 to 10). Pairs combining a control 50% oligosaccharide sample and a 50% oligosaccharide sample containing ERG at each concentration were presented, and the panel evaluated which sample of the presented pair was perceived to be sweeter by a paired comparison test. The test results are shown in Table 15.

**[Table 15]**

| | | Panelist | | |
|---|---|---|---|---|
| Control | Test product | A | B | C |
| ERG0 | ERG0 | ◊ | ◊ | ◊ |
| ERG0 | ERG0.5 | ○ | ○ | ○ |
| ERG0 | ERG1 | ○ | ○ | ○ |
| ERG0 | ERG3 | ○ | ○ | ○ |
| ERG0 | ERG5 | ○ | ○ | ○ |
| ERG0 | ERG10 | ○ | ○ | ○ |
| ERG0 | ERG20 | ○ | ○ | ○ |

For the 50% oligosaccharide samples to which 0.5 ppm or more of ERG was added, all the panelists evaluated that the sweetness was enhanced.

### (5) Aspartame

Samples were prepared by adding ERG to a 10% aspartame sample of aspartame (Pal Sweet Slim Up Sugar, Ajinomoto Co., Inc.) to the ERG concentrations shown in Table 16 (ERG0 to 20). Pairs combining a control 10% aspartame sample and a 10% aspartame sample containing ERG at each concentration were presented, and the panel evaluated which sample of the presented pair was perceived to be sweeter by a paired comparison test. The test results are shown in Table 16.

**[Table 16]**

| | | Panelist | | | |
|---|---|---|---|---|---|
| Control | Test product | A | B | C | D |
| ERG0 | ERG0 | ◊ | ◊ | ◊ | ◊ |
| ERG0 | ERG0.5 | ○ | ○ | ◊ | ○ |
| ERG0 | ERG1 | ○ | ○ | ○ | ○ |
| ERG0 | ERG3 | ○ | ○ | ○ | ○ |
| ERG0 | ERG5 | ○ | ○ | ○ | ○ |
| ERG0 | ERG10 | ○ | ○ | ○ | ○ |
| ERG0 | ERG20 | ○ | ○ | ○ | ○ |

For the 10% aspartame samples to which 0.5 ppm or more of ERG was added, three panelists evaluated that the sweetness was enhanced. This corresponds to 0.0005% by weight or more of ERG having a sweetness-enhancing effect, based on the aspartame content of the food or beverage product.

### (6) Stevia

Samples were prepared by adding ERG to a 0.1% stevia sample to the ERG concentrations shown in Table 17 (ERG0 to 20). Pairs combining a control 0.1% stevia sample and a 0.1% stevia sample containing ERG at each concentration were presented, and the panel evaluated which sample of the presented pair was perceived to be sweeter by a paired comparison test. The test results are shown in Table 17.

**[Table 17]**

| | | Panelist | | |
|---|---|---|---|---|
| Control | Test product | A | B | C |
| ERG0 | ERG0 | ◇ | ◇ | ◇ |
| ERG0 | ERG0.5 | ○ | ○ | ○ |
| ERG0 | ERG1 | ○ | ○ | ○ |
| ERG0 | ERG3 | ○ | ○ | ○ |
| ERG0 | ERG5 | ○ | ○ | ○ |
| ERG0 | ERG10 | ○ | ○ | ○ |
| ERG0 | ERG20 | ○ | ○ | ○ |

For the 0.1% stevia samples to which 0.5 ppm or more of ERG was added, all the panelists evaluated that the sweetness was enhanced. This corresponds to 0.05% by weight or more of ERG having a sweetness-enhancing effect, based on the stevia content of the food or beverage product.

### (7) Amino acid (glycine)

Samples were prepared by adding ERG to a 12.5% glycine sample to the ERG concentrations shown in Table 18 (ERG0 to 10). Pairs combining a control 12.5% glycine sample and a 12.5% glycine sample containing ERG at each concentration were presented, and the panel evaluated which sample of the presented pair was perceived to be sweeter by a paired comparison test. The test results are shown in Table 18.

**[Table 18]**

| | | Panelist | | |
|---|---|---|---|---|
| Control | Test product | A | B | C |
| ERG0 | ERG0 | ◇ | ◇ | ◇ |
| ERG0 | ERG0.5 | ○ | ○ | ○ |
| ERG0 | ERG1 | ○ | ○ | ○ |
| ERG0 | ERG3 | ○ | ○ | ○ |
| ERG0 | ERG5 | ○ | ○ | ○ |
| ERG0 | ERG10 | ○ | ○ | ○ |
| ERG0 | ERG20 | ○ | ○ | ○ |

For the 12.5% glycine samples to which 0.5 ppm or more of ERG was added, all the panelists evaluated that the sweetness was enhanced. This corresponds to 0.0004% by weight or more of ERG having a sweetness-enhancing effect, based on the glycine content of the food or beverage product.

### [Test 10: Sweetness-enhancing effect of amazake]

### Production of amazake

1,250 g of white rice was soaked in water overnight, transferred to a colander, and drained for 2 hours. Thereafter, the rice was wrapped in a Tetoron cloth and steamed at 100°C for 40 minutes. The steamed rice was cooled to 45°C, and a seed koji was inoculated. The rice was wrapped in a Pylen cloth, and koji-making was started under the conditions of 35°C and 95% humidity. 19 hours after the start of koji-making, the rice koji was loosened and wrapped again in the Pylen cloth. When the product temperature reached 40°C, the Pylen cloth was spread and the rice koji was leveled. After another 40 hours, the rice koji was well loosened and left to stand again. After 42 hours, the koji was taken out. To 800 g of the rice koji, a 2.5-fold amount of hot water at 60°C was added, and a saccharification reaction was carried out at 55°C and 200 rpm. After 16 hours, Brix was adjusted to 18%, and the mixture was transferred to a heat-resistant container and sterilized at 85°C for 30 minutes. When adjusting Brix of the amazake, water or ergothioneine was added to adjust the ergothioneine concentration in the amazake to 12 ppm (water added), 30 ppm, 45 ppm, 80 ppm, 145 ppm, and 345 ppm.

The sweetness of the amazake was sensorially evaluated by an expert panel consisting of five trained sweetness-sensitive subjects.

Pairs combining amazake containing ERG at different concentrations shown in Table 19 (ERG12 to 345) were presented to the expert panel of five, and the panelists evaluated which sample of the presented pair was perceived to be sweeter by a paired comparison test.

The results are shown in Table 19. For the amazake prepared to have ERG of 30 ppm or more, all the panelists evaluated that the sweetness was enhanced. For the amazake prepared to have ERG of 345 ppm, a majority of the panelists evaluated that the degree of sweetness was equivalent to that of the amazake prepared to have ERG of 145 ppm, and the degree of sweetness enhancement did not change from that to which 145 ppm was added.

For the amazake, it was evaluated that, when ERG was contained at 30 ppm to 145 ppm, the sweetness was sequentially enhanced as the content increased. Even when it exceeded 145 ppm, the sweetness-enhancing effect was no different from that in the case of adding 145 ppm.

In addition, when the amazake was produced under the same conditions as in Test 6 using an Aspergillus oryzae strain, which is a high ergothioneine-producing koji mold strain, in which a G428S mutation was introduced into the AO090005000664 ortholog gene, the ERG content of the obtained amazake was 115 ppm. Not only was the sweetness enhanced, but also the aroma was favorable due to reduced koji odor, and the mouthfeel was smoother because fewer particles had large particle diameters.

**[Table 19]**

| | | Panelist | | | | |
|---|---|---|---|---|---|---|
| Control | Test product | A | B | C | D | E |
| ERG12 | ERG30 | ○ | ○ | ○ | ○ | ○ |
| ERG30 | ERG45 | ○ | ○ | ○ | ○ | ○ |
| ERG45 | ERG80 | ○ | ○ | ○ | ○ | ○ |
| ERG80 | ERG145 | ○ | ○ | ○ | ○ | ○ |
| ERG145 | ERG345 | ◊ | ◊ | ○ | ○ | ◊ |

### [Test 11: Sweetness-enhancing effect of mirin]

Commercially available mirin (ergothioneine content 1.6 ppm) was heated to remove alcohol, and ergothioneine was added thereto so that the final concentrations became 1.6 ppm, 2.1 ppm, 4.6 ppm, 6.6 ppm, 11.6 ppm, and 21.6 ppm (ERG1.6 to 21.6) to prepare mirins.

For the mirins to which ERG was added at each concentration (the concentration was not disclosed to the panel), a drinking test by an expert panel of three was conducted using mirin to which no ergothioneine was added (ERG1.6) as a control, and the sweetness was sensorially evaluated.

Pairs combining the control mirin and the mirin containing ergothioneine at each concentration were presented to the expert panel, and the panel evaluated which sample of the presented pair was perceived to be sweeter by a paired comparison test. The results are shown in Table 20.

**[Table 20]**

| | | Panelist | | |
|---|---|---|---|---|
| Control | Test product | A | B | C |
| ERG1.6 | ERG1.6 | ◇ | ◇ | ◇ |
| ERG1.6 | ERG2.1 | ○ | ○ | ○ |
| ERG1.6 | ERG2.6 | ○ | ○ | ○ |
| ERG1.6 | ERG4.6 | ○ | ○ | ○ |
| ERG1.6 | ERG6.6 | ○ | ○ | ○ |
| ERG1.6 | ERG11.6 | ○ | ○ | ○ |
| ERG1.6 | ERG21.6 | ○ | ○ | ○ |

All the panelists evaluated not only the 2.1 ppm mirin to which 0.5 ppm of ERG was added, but also the mirin containing 4.6 to 21.6 ppm of ERG, as having enhanced sweetness.

### [Test 12: Production of mirin]

After raw material processing such as washing with water, soaking in water, draining, steaming, and allowing to cool was performed on polished rice by an ordinary method, 0.1% of seed koji by weight of rice was inoculated and mixed thereto, and koji-making was managed in a thermo-hygrostat at an optimum koji-making temperature of 32°C to 38°C to obtain koji. As the seed koji, an Aspergillus oryzae strain (Example) in which a G428S mutation was introduced into the AO090005000664 ortholog gene and a wild strain thereof (Comparative Example) were respectively used. According to an ordinary method for producing mirin, 48 g of the above-described rice koji (approximately 40 g as raw polished non-glutinous rice) was placed in a preparation container, and mixed with 290 g of glutinous rice (kakemai) that had been washed, soaked in water, drained, steamed, and allowed to cool by an ordinary method. Next, 155 ml of neutral alcohol (alcohol content: 35% (v/v)) was added, and saccharification was carried out at 30°C for 1 month to obtain an aged mirin moromi. Subsequently, the aged mirin moromi was pressed by an ordinary method to obtain a crude mirin filtrate. The crude mirin filtrate was heat-sterilized at a reaching temperature of 90°C, and then clarified and filtered to obtain mirin.

The concentration of ERG in the mirin obtained by the clarification and filtration was 11 ppm in the Example and 2 ppm in the Comparative Example.

The mirin obtained in Example had enhanced not only sweetness but also umami as compared to that of Comparative Example.

### [Test 13: Sweetness-enhancing effect of soy sauce]

### (1) Specially selected whole soybean soy sauce

Soy sauces were prepared by adding ergothioneine to a commercially available specially selected whole soybean soy sauce (Kikkoman Corporation) to addition concentrations of 10 ppm, 20 ppm, and 50 ppm (EGR10, 20, 50).

A drinking test by a panel of three experts was conducted on the soy sauce to which ERG was added at each concentration (the concentration was not disclosed to the panel), using a soy sauce to which no ergothioneine was added (ERG0) as a control, and the sweetness was sensorially evaluated by a paired comparison test. The results are shown in Table 21.

**[Table 21]**

| | | Panelist | | |
|---|---|---|---|---|
| Control | Test product | A | B | C |
| ERG0 | ERG0 | ◇ | ◇ | ◇ |
| ERG0 | ERG10 | ○ | ○ | ○ |
| ERG0 | ERG20 | ○ | ○ | ○ |
| ERG0 | ERG50 | ○ | ○ | ○ |

Regarding the soy sauce to which 10 ppm or more of ERG was added, all the panelists evaluated that the sweetness was enhanced.

### (2) Amakuchi soy sauce

The same test as in (1) described above was conducted by adding ERG to a commercially available amakuchi soy sauce (Golden Murasaki, Fundokin Shoyu). The results are shown in Table 22.

**[Table 22]**

| | | Panelist | | |
|---|---|---|---|---|
| Control | Test product | A | B | C |
| ERG0 | ERG0 | ◇ | ◇ | ◇ |
| ERG0 | ERG10 | ○ | ○ | ○ |
| ERG0 | ERG20 | ○ | ○ | ○ |
| ERG0 | ERG50 | ○ | ○ | ○ |

Regarding the soy sauce to which 10 ppm or more of ERG was added, all the panelists evaluated that the sweetness was enhanced.

### [Test 14: Sweetness-enhancing effect of prepared soy milk]

Samples of prepared soy milk (EGR0.5 to 20) were prepared by adding ergothioneine to commercially available prepared soy milk (Kikkoman Corporation) to final concentrations of 0.5 ppm, 1 ppm, 3 ppm, 5 ppm, 10 ppm, and 20 ppm.

A drinking test by a panel of three experts was conducted on the prepared soy milk to which ERG was added at each concentration (the concentration was not disclosed to the panel), using a prepared soy milk to which no ergothioneine was added (ERG0) as a control, and the sweetness was sensorially evaluated by a paired comparison test. The results are shown in Table 23.

**[Table 23]**

| | | Panelist | | |
|---|---|---|---|---|
| Control | Test product | A | B | C |
| ERG0 | ERG0 | ◇ | ◇ | ◇ |
| ERG0 | ERG0.5 | ◇ | ◇ | ◇ |
| ERG0 | ERG1 | ◇ | ○ | ◇ |
| ERG0 | ERG3 | ○ | ○ | ◇ |
| ERG0 | ERG5 | ○ | ○ | ○ |
| ERG0 | ERG10 | ○ | ○ | ○ |
| ERG0 | ERG20 | ○ | ○ | ○ |

For the prepared soy milk containing 3 ppm of ERG, two panelists evaluated that the sweetness was enhanced, and for the prepared soy milk containing 5 ppm of ERG, all the panelists evaluated the enhancement of sweetness. In addition, the prepared soy milk containing 40 ppm of ERG had an increased creamy feel.

### [Test 15: Sweetness-enhancing effect of milk]

Samples of milk (EGR0.5 to 20) were prepared by adding ergothioneine to commercially available milk to final concentrations of 0.5 ppm, 1 ppm, 3 ppm, 5 ppm, 10 ppm, and 20 ppm.

A drinking test by a panel of three experts was conducted on the milk to which ERG was added at each concentration (the concentration was not disclosed to the panel), using a milk to which no ergothioneine was added (ERG0) as a control, and the sweetness was sensorially evaluated by a paired comparison test. The results are shown in Table 24.

**[Table 24]**

| | | Panelist | | |
|---|---|---|---|---|
| Control | Test product | A | B | C |
| ERG0 | ERG0 | ◇ | ◇ | ◇ |
| ERG0 | ERG0.5 | ◇ | ◇ | ◇ |
| ERG0 | ERG1 | ◇ | ○ | ◇ |
| ERG0 | ERG3 | ○ | ○ | ◇ |
| ERG0 | ERG5 | ○ | ○ | ○ |
| ERG0 | ERG10 | ○ | ○ | ○ |
| ERG0 | ERG20 | ○ | ○ | ○ |

For the milk containing 5 ppm of ERG, all the panelists evaluated that the sweetness was enhanced.

### [SEQUENCE LISTING]

PCT24359KI.xml

## Claims

1. A sweetness enhancer comprising ergothioneine as an active ingredient.

2. A sweetness enhancer for a food or beverage product containing a sugar-based sweet component, comprising ergothioneine as an active ingredient.

3. The sweetness enhancer according to Claim 2, wherein the sugar-based sweet component is one or more selected from the group consisting of sucrose (sugar), glucose (dextrose), fructose, fructose-glucose syrup, oligosaccharides, starch syrup, trehalose, maltose, and sugar alcohols such as sorbitol, mannitol, and xylitol.

4. A food or beverage product having enhanced sweetness and containing a sugar-based sweet component, in which the food or beverage product contains ergothioneine in an amount of 0.0001% by weight or more relative to a content of sucrose, 0.0001% by weight or more relative to a content of glucose, or 0.0003% by weight or more relative to a content of fructose.

5. The food or beverage product according to Claim 4, wherein the food or beverage product is a food, a beverage, or a seasoning.

6. The food or beverage product according to Claim 4, wherein the food or beverage product is liquid sugar, prepared soy milk, milk, or an alcoholic beverage.

7. The food or beverage product according to Claim 6, wherein the alcoholic beverage is sake, shochu, mirin, or amazake obtained by saccharification and/or brewing of raw grains using koji.

8. The food or beverage product according to Claim 4, wherein the food or beverage product is produced using a high ergothioneine-producing koji mold strain.

9. An amazake comprising 30 ppm or more of ergothioneine.

10. A mirin comprising 2.5 ppm or more of ergothioneine.

11. A sweetness enhancer for a food or beverage product containing a non-sugar sweet component, comprising ergothioneine as an active ingredient.

12. The sweetness enhancer according to Claim 11, wherein the non-sugar sweet component is one or more selected from the group consisting of high-intensity sweeteners including aspartame, acesulfame potassium, sucralose, stevia, luo han guo, licorice, and sweet amino acids.

13. A food or beverage product having enhanced sweetness and containing a non-sugar sweet component, comprising ergothioneine in an amount of 0.0005% by weight or more relative to a content of a high-intensity sweetener or 0.0004% by weight or more relative to a content of a sweet amino acid.

14. A method for enhancing sweetness of a food or beverage product, comprising incorporating ergothioneine into the food or beverage product.
